# EUROPEAN PATENT SPECIFICATION

(11) **EP 3 769 793 B1**
(45) Date of publication and mention of the grant of the patent: **06.09.2023**
(21) Application number: 20185560.8
(22) Date of filing: 13.07.2020
(51) Int. Cl.: A61L 2/22, B29C 49/64, B65B 55/04, B67C 7/00

(54) **METHOD FOR PRODUCING STERILE RECEPTACLES AND BOTTLING PLANT COMPRISING SAID PRODUCING APPARATUS**
VERFAHREN ZUR HERSTELLUNG STERILER BEHÄLTER UND ABFÜLLANLAGE MIT DIESER HERSTELLUNGSVORRICHTUNG
PROCEDE DE PRODUCTION DE RECIPIENTS STERILES ET INSTALLATION D'EMBOUTEILLAGE COMPRENANT LEDIT APPAREIL DE PRODUCTION

(30) Priority: 26.07.2019 IT 201900013053
(43) Date of publication of application: 27.01.2021
(73) Proprietor: Gea Procomac S.p.A., 43038 Sala Baganza (PR) (IT)
(72) Inventor: CASAPPA, Luigi, 43038 SALA BAGANZA (PR) (IT); ABELLI, Paolo, 43038 SALA BAGANZA (PR) (IT)
(74) Representative: Dondi, Silvia

(56) References cited:
- US-A1- 2010 047 120
- US-A1- 2011 272 861
- US-A1- 2012 042 611

## Description

The present invention relates to a method for producing sterile receptacles.

The reference sector is the bottling of so-called "sensitive" food products, that is, products that are particularly sensitive to bacteriological contamination and oxidation, such as, for example, isotonic drinks, juices, nectars, soft drinks, tea, milk-based drinks, coffee-based drinks, etc., for which the prevention of possible microbiological contamination throughout all packaging stages is of fundamental importance.

Packaging lines using aseptic technology are already known in the prior art, in which the various operations take place in a contaminationcontrolled environment, so that the bottled products can be stored for a prolonged period of time and have chemical/physical and organoleptic stability even at room temperature.

Aside from differences in design, a "conventional" aseptic bottling line includes:
- forming the container starting with a parison made of a thermoplastic material;
- chemical sterilization of the formed container;
- rinsing, filling and capping of the filled container, to be carried out in a sterile environment.

The main drawback of conventional lines is related to the need to have to sterilize the container once it has been formed and to maintain the sterilized state thereof throughout all subsequent operations, for example the filling and capping operations.

A modern concept of an aseptic bottling line instead includes:
- sterilization of the parison using chemical agents or radiations;
- "aseptic" forming of the container starting with a sterilized parison;
- filling and capping of the filled container, to be carried out in a sterile environment.

In an aseptic line, container and closure sterilization performance is expressed by the number of decimal reductions that the sterilization treatment is capable of achieving against a reference microorganism. For example, in aseptic lines that package low-acid products, six decimal reductions are generally required, whereas in lines that treat high-acid products, four decimal reductions are sufficient.

The main disadvantage of a "modern" line is related to the need to maintain high sterilization performance in all units in order to ensure the required decimal reductions, due to the lack of contamination control on all steps upstream of the aseptic bottling line (parisons production in dedicated factories, transport to the bottling factory, storage and feeding of the forming machine).

However, ensuring four or six decimal reductions substantially affects the complexity of the overall dimensions of the aseptic line and also increases its operating costs.

For these reasons, in recent years some manufacturers have developed solutions which provide for the production of parisons upstream of the packaging line, in the same factory.

For example, document EP2578504 shows an aseptic filling system in which all operating units, comprising the parison moulding press, are placed in a clean chamber. Each operating unit is also contained in a dedicated cabin at a higher pressure than the pressure of the clean chamber in order to ensure a higher degree of purity within the cabin itself. The operator is allowed to access to each cabin of the line.

Similar solutions are also described in documents US2011/0219728 (see in particular the embodiment illustrated in figure 2 of such document), EP0794903 and EP2324987.

In the latter, explicit reference is made to the need to sterilize the internal surfaces of the clean chamber and the external surfaces of the cabins/boxes containing the individual operating units.

The main disadvantage of the solutions integrating the parison moulding press within the aseptic line is related to the increase in volumes and surfaces to be sterilized before installation and to the increase in volumes to be kept sterile during production.

All the sterility maintenance and line management activities are thereby complicated. In addition, the cleaning and sterilization times are lengthened.

Another disadvantage is due to the increased complexity of the press in order to be compatible with the production rate of the downstream units. For example, document EP2578504 provides a temporary storage buffer of the parisons between the press and the blowing machine.

In document WO 2016/088003, in the name of the Applicant, a bottling system is proposed which includes moulding the parisons, forming and filling the receptacles in the same line. The parisons are sterilized in the receptacle forming unit using plasma.

This solution increases the constructive complexity of the aseptic blowing machine and makes maintenance more difficult, having to always access the blowing machine to repair failures related to the sterilization devices.

In this context, the technical task underpinning the present invention is to provide a method for producing sterile receptacles, which obviates the drawbacks of the prior art cited above.

In particular, an object of the present invention is to provide a method for producing a sterile receptacle in which the sterilization times of the receptacle itself are shortened, the operations for maintaining the sterility are simplified and therefore the operating costs are reduced.

The stated technical task and specified objects are substantially achieved by a method for producing a sterile receptacle according to the claims.

Further characteristics and advantages of the present invention will more fully emerge from the non-limiting description of a preferred but not exclusive embodiment of a method for producing sterile receptacles, as illustrated in the accompanying drawings, in which:

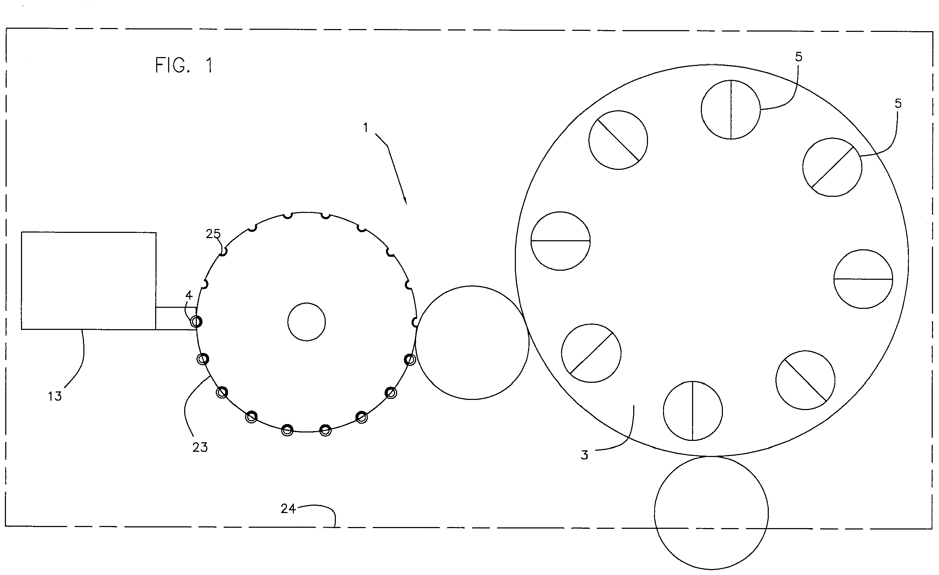

- figure 1 illustrates an apparatus for producing sterile receptacles, according to the claimed method, in a schematic plan view;
- figure 2 illustrates a detail of the transferring unit of the producing apparatus of figure 1, in a lateral view;
- figure 3 schematically illustrates a parison, in a lateral view;
- figure 4 illustrates a variant of the producing apparatus of figure 1. With reference to the figures, the number 1 indicates an apparatus for producing sterile receptacles starting from parisons 4 made of thermoplastic material.

Preferably, each parison 4 has a tubular body 4a and a neck 4b (usually threaded) not subject to processing.

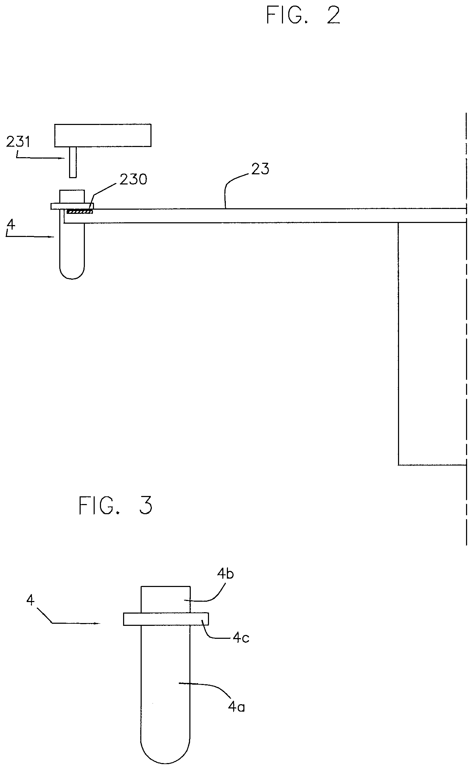

Figure 3 schematically illustrates the structure of a parison 4, in which an annular protrusion of the neck 4b, indicated by reference 4c, is also visible. This annular protrusion 4c is known in the art as a "bague".

The producing apparatus 1 comprises:
- a moulding unit 13 for moulding parisons 4 starting from granules of thermoplastic material (e.g. PET);
- a forming unit 3 for forming the moulded parisons 4, in particular by stretch-blowing.

A transferring unit 23 is interposed between the moulding unit 13 and the forming unit 3, which comprises at least one thermal conditioning device 230 configured to heat the tubular portion 4a of the parisons 4 in such a way that the parisons 4 leaving the transferring unit 23 have a thermal profile adapted to allow forming by stretch-blowing in the forming unit 3.

In fact, the parisons 4 leave the moulding unit 13 with the tubular body 4a at a temperature above 100°C.

The neck 4b is instead cooled so as to have a temperature lower than the glass temperature of the PET, preferably between 20°C and 40°C.

This creates a temperature imbalance between the neck 4b and the tubular body 4a, which imbalance generates an uncontrolled thermal profile which extends down to below the bague 4c. This situation is not suitable for forming the parison 4.

Thanks to the passage in the transferring unit 23 provided with the thermal conditioning device 230, the parisons 4 re-establish an optimal thermal profile adapted to allow forming by stretch-blowing.

Sterilization devices 231 of the parisons 4 are located on the transferring unit 23 which are configured to sterilize the parisons 4 at least internally, i.e., the walls inside the tubular cavity.

Advantageously, therefore, both the thermal conditioning of the parisons 4 and their sterilization occur in the transferring unit 23.

In accordance with the embodiment described and illustrated herein, the transferring unit 23 is of the rotary carousel type.

In the embodiment described and illustrated herein, the transferring unit 23 comprises a star-wheel having a plurality of supporting stations for supporting the parisons 4 by the neck 4b thereof.

Preferably, each supporting station comprises a pocket 25 at the external circumference of the star-wheel for receiving and supporting the neck 4b of one of the parisons 4.

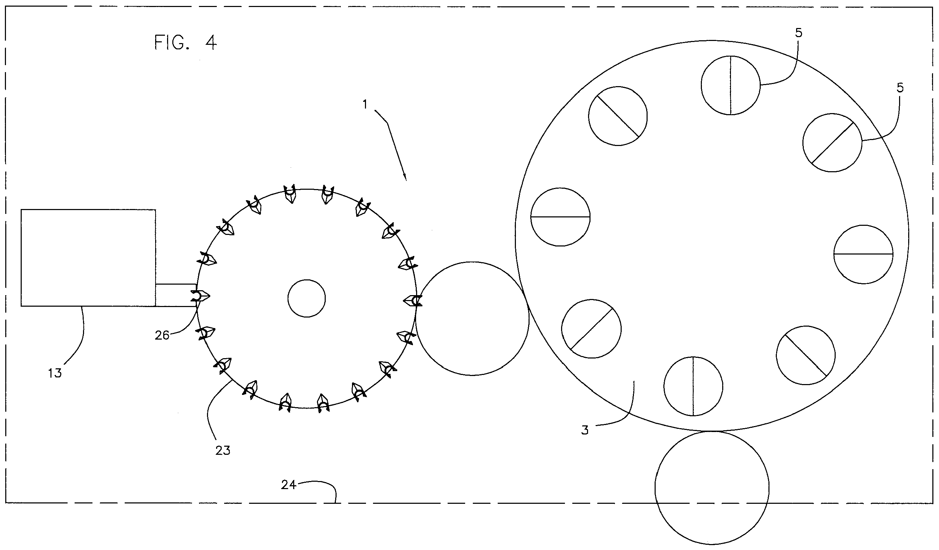

As an alternative to the pocket, in each supporting station gripping means 26 (e.g., grippers) of the neck 4b of the parison 4 is present. This variant is illustrated in figure 4.

According to the invention, a thermal conditioning device 230 is arranged at each supporting station.

In accordance with the embodiment described and illustrated herein, each thermal conditioning device 230 is a heating element, such as a thermal resistance, installed in the corresponding pocket 25.

In particular, the thermal conditioning device 230 is arranged in such a way as to contact a lower portion of the bague 4c of the parison 4 present in the pocket 25, as seen in figure 2.

The thermal conditioning device 230 may be configured at least in one activated condition in which it heats a zone of the tubular body 4a beneath the bague 4c of the parison 4.

In one embodiment, the thermal conditioning device 230 is an infrared or microwave type generator, preferably mounted on the star-wheel of the transferring unit 23.

One of the sterilization devices 231 is also located at each supporting station (hence the pocket 25 or the gripping means 26).

For example, each sterilization device 231 consists of a spray nozzle of a sterilizing fluid.

In one embodiment not according to the invention the transferring unit 23 is of the linear type.

The moulding unit 13 and the forming unit 3 are synchronized so that each moulded parison 4 is transferred directly to the forming unit 3 via the transferring unit 23.

Preferably, the moulding unit 13 of the parisons 4 comprises a plurality of moulding stations, in each of which a mould is arranged constituted by a concave portion (also called the "female mould") and a convex portion (also called the "male mould") which is insertable in the concave portion. Preferably, the moulding unit 13 is a conventional type, alternating cycle machine with injection-type moulds. Alternatively, the moulding unit 13 is a continuously operating rotary machine with compression or injection-compression type moulds.

In accordance with one embodiment, the moulding unit 13 comprises means for cleaning the surfaces of the moulds adapted to come into contact with the parisons 4.

According to the invention, the moulding unit 13 is arranged in an ultra-clean environment 24. In this context, ultra-clean environment is intended as a volume which is separated from the (dirty) external environment by means of a physical separation which has the purpose of limiting the entrance of contaminants from the external environment.

In particular, the physical separation need not necessarily be tight sealed. According to the invention, the ultra-clean environment 24 is maintained at an overpressure value of about 10 Pa. Preferably, microfiltered air flows from special devices (HEPA filters, e.g., level H13) used to generate such overpressure. The purpose of the ultra-clean environment 24 is to limit the contamination of the parisons 4.

According to the invention, the thermal conditioning unit 23 and the forming unit 3 are also located in the ultra-clean environment 24.

Preferably, the forming unit 3 is of the rotary carousel type. In an alternative embodiment (not shown), the forming unit 3 is of the linear type.

The forming unit 3 comprises a plurality of forming stations 5 in each of which a mould of a known type is arranged.

The proposed disclosure further relates to a bottling plant not according to the invention comprising:
- the producing apparatus 1 for producing sterile receptacles described above;
- a filling apparatus for filling the formed receptacles, arranged downstream of the producing apparatus 1;
- a closing apparatus for closing the filled receptacles, which is arranged downstream of the filling apparatus.

The method for producing sterile receptacles, in accordance with the present invention, is described below.

First of all, a step is provided for moulding the parisons 4 starting from granules of thermoplastic material.

Preferably, the surfaces of the moulds adapted to come into contact with the parisons 4 are cleaned before starting the moulding.

Subsequently, the granules are melted and moulded in the moulds by injection or compression or injection-compression moulding.

At the end of the moulding step the parisons 4 have a temperature around 100°C. The neck 4b of the parisons 4 is cooled to allow it to be gripped. The moulded parisons 4 are then subjected to a thermal conditioning step to give the parisons 4 a predefined thermal profile adapted to allow the forming by stretch-blowing.

The moulded parisons 4 are also subjected to a sterilization step.

Originally, the sterilization step overlaps at least partly with the thermal conditioning step.

In accordance with a variant of the method, the sterilization step of the parisons 4 and the thermal conditioning step are simultaneous, i.e., completely overlapping.

At the end of the sterilization/thermal conditioning step, the parisons 4 are introduced into the moulds of the forming unit 3 and the receptacles are formed by stretch-blowing.

Leaving the forming unit 3, the receptacles are transferred to the filling apparatus.

In particular, the neck 4b of the receptacles (which has not undergone processing) is maintained in sterile conditions thanks to the presence of a first isolation device, which in fact isolates the volume in which the filling occurs - volume having an extension such as to comprise the neck 4b of the receptacles and the filling nozzles but not the body of the formed receptacles.

Once filled, the receptacles are sent to the closing or capping apparatus. The characteristics of the method for producing sterile receptacles according to the present invention emerge clearly from the above description, as do the advantages.

First of all, having integrated the parison moulding unit within the receptacle producing apparatus, together with the fact that the sterilization step of the parisons is at least overlapping the thermal conditioning step before the forming by stretch-blowing, allows reducing the structural complexity of the "aseptic" blowing machine with isolator and of all the parts operating at the interface thereof (e.g., sealing systems between the sterile zone and the external environment, separation of the stretching rod, sterilization system of the blowing air circuit, etc.).

In fact, the integration of the moulding unit allows to keep the contamination of the parisons under control during their generation and to deliver parisons to the blowing machine with a low level of contamination.

This is also due to the high temperature of the parisons leaving the moulding unit, which is about 100°C.

The handling of the parisons only by means of gripping shells operating on the neck thereof and the maintenance of the parisons in an ultra-clean environment also contributes to this end.

Furthermore, having disconnected the sterilization from the blowing machine facilitates maintenance and, in particular, the repair of failures related to the sterilization devices.

## Claims

1. Method for producing sterile receptacles, comprising the steps of:
- obtaining parisons (4) by melting and moulding granules of thermoplastic material in a moulding unit (13) for moulding parisons (4), each of said parisons (4) having a tubular body (4a) and a neck (4b) with an annular protrusion called bague (4c), the parisons (4) leaving the moulding unit (13) with the tubular body (4a) at a temperature above 100°C and the neck (4b) being cooled so as to have a temperature between 20°C and 40°C, this creating a temperature imbalance between the neck (4b) and the tubular body (4a), which temperature imbalance generates an uncontrolled thermal profile which extends down below the bague (4c);
- sterilizing the moulded parisons (4);
- submitting the moulded parisons (4) to thermal conditioning in a transferring unit (23) comprising a star-wheel having a plurality of supporting stations for supporting the parisons (4) by their neck (4b), in each supporting station being arranged one thermal conditioning device (230) configured to heat a tubular body (4a) of said parisons (4) so that said parisons (4) leaving the transferring unit (23) have a predefined thermal profile adapted to allow forming by stretch-blowing;
- obtaining the receptacles by stretch-blowing said parisons (4) in a forming unit (3) for stretch-blowing the parisons (4), said transferring unit (23) being interposed between said moulding unit (13) and said forming unit (3),
said moulding unit (13), said forming unit (3) and said transferring unit (23) being arranged in an ultra-clean environment (24), i.e. in a volume that is separated from the external environment by means of a physical separation that has the purpose of limiting the entrance of contaminants from the external environment, said ultra-clean environment (24) being maintained at an overpressure value of about 10 Pa, microfiltered air flows coming from HEPA filters of level H13 being used to generate such overpressure,
the step of sterilizing the moulded parisons (4) occurring at least partly during the step of thermal conditioning of the parisons (4) in a plurality of sterilization devices (231) configured to sterilize at least internally said parisons (4), said sterilization devices (231) being installed on said transferring unit (23), one of the sterilization devices (231) being located at each supporting station.

2. Method according to claim 1, wherein the step of sterilizing the parisons (4) and the step of thermal conditioning of the parisons (4) are simultaneous.

## Patentansprüche

1. Verfahren zur Herstellung steriler Behälter, umfassend die folgenden Schritte:
- Erhalten von Vorformlingen (4) durch Schmelzen und Formen von Granulat aus thermoplastischem Material in einer Formeinheit (13) zum Formen von Vorformlingen (4), wobei ein jeder der Vorformlinge (4) einen rohrförmigen Körper (4a) und einen Hals (4b) mit einem ringförmigen Vorsprung, der als Ring (4c) bezeichnet wird, aufweist, wobei die Vorformlinge (4) die Formeinheit (13) mit dem rohrförmigen Körper (4a) bei einer Temperatur über 100 °C verlassen und der Hals (4b) gekühlt wird, um eine Temperatur zwischen 20 °C und 40 °C aufzuweisen, wodurch ein Temperaturungleichgewicht zwischen dem Hals (4b) und dem rohrförmigen Körper (4a) erzeugt wird, wobei das Temperaturungleichgewicht ein unkontrolliertes Wärmeprofil erzeugt, das sich unterhalb des Rings (4c) erstreckt;
- Sterilisieren der geformten Vorformlinge (4);
- Unterziehen der geformten Vorformlinge (4) einer thermischen Konditionierung in einer Transfereinheit (23), die ein Sternrad mit einer Vielzahl von Stützstationen zum Stützen der Vorformlinge (4) an ihrem Hals (4b) umfasst, wobei in einer jeden Stützstation eine Vorrichtung zur thermischen Konditionierung (230) angeordnet ist, die ausgelegt ist, um einen rohrförmigen Körper (4a) der Vorformlinge (4) zu erwärmen, so dass die Vorformlinge (4), die die Transfereinheit (23) verlassen, ein vordefiniertes thermisches Profil aufweisen, das angepasst ist, um die Formierung durch Streckblasen zu ermöglichen;
- Erhalten der Behälter durch Streckblasen der Vorformlinge (4) in einer Formungseinheit (3) zum Streckblasen der Vorformlinge (4), wobei die Transfereinheit (23) zwischen der Formeinheit (13) und der Formungseinheit (3) angeordnet ist,
wobei die Formeinheit (13), die Formungseinheit (3) und die Transfereinheit (23) in einer ultrareinen Umgebung (24) angeordnet sind, d. h. in einem Volumen, das von der äußeren Umgebung mittels einer physischen Trennung getrennt ist, deren Funktion ist, den Eintritt von Verunreinigungen aus der äußeren Umgebung zu begrenzen, wobei die ultrareine Umgebung (24) auf einem Überdruckwert von etwa 10 Pa gehalten wird, wobei mikrogefilterte Luftströme, die von HEPA-Filtern des Niveaus H13 kommen, verwendet werden, um einen solchen Überdruck zu erzeugen,
wobei der Schritt zum Sterilisieren der geformten Vorformlinge (4) zumindest teilweise während des Schritts zum thermischen Konditionieren der Vorformlinge (4) in einer Vielzahl von Sterilisationsvorrichtungen (231) erfolgt, die ausgelegt sind, um die Vorformlinge (4) zumindest intern zu sterilisieren, wobei die Sterilisationsvorrichtungen (231) an der Transfereinheit (23) installiert sind, wobei eine der Sterilisationsvorrichtungen (231) sich an jeder Stützstation befinden.

2. Verfahren nach Anspruch 1, wobei der Schritt zum Sterilisieren der Vorformlinge (4) und der Schritt zum thermischen Konditionieren der Vorformlinge (4) gleichzeitig erfolgen.

## Revendications

1. Procédé de production de récipients stériles, comprenant les étapes de :
- obtenir des paraisons (4) par fusion et moulage de granulés de matière thermoplastique dans une unité de moulage (13) pour mouler des paraisons (4), chacune desdites paraisons (4) comportant un corps tubulaire (4a) et un col (4b) avec une protubérance annulaire appelée bague (4c), les paraisons (4) quittant l'unité de moulage (13) avec le corps tubulaire (4a) à une température supérieure à 100 °C et le col (4b) étant refroidi de manière à avoir une température comprise entre 20 et 40 °C, ceci créant un déséquilibre de température entre le col (4b) et le corps tubulaire (4a), ce déséquilibre de température génère un profil thermique incontrôlé qui se prolonge jusqu'en dessous de la bague (4c) ;
- stériliser les paraisons moulés (4) ;
- soumettre les paraisons moulés (4) à un conditionnement thermique dans une unité de transfert (23) comprenant une étoile comportant une pluralité de postes de support pour supporter les paraisons (4) par leur col (4b), dans chaque poste de support étant disposé un dispositif de conditionnement thermique (230) configuré pour chauffer un corps tubulaire (4a) desdites paraisons (4) de sorte que lesdites paraisons (4) quittant l'unité de transfert (23) aient un profil thermique prédéfini adapté pour permettre le formage par étirage-soufflage ;
- obtenir les récipients par étirage-soufflage desdites paraisons (4) dans une unité de formage (3) pour l'étirage-soufflage des paraisons (4), ladite unité de transfert (23) étant interposée entre ladite unité de moulage (13) et ladite unité de formage (3),
ladite unité de moulage (13), ladite unité de formage (3) et ladite unité de transfert (23) étant disposées dans un environnement ultranettoyé (24), c'est-à-dire dans un volume étant séparé de l'environnement extérieur au moyen d'une séparation physique ayant pour but de limiter l'entrée de contaminants provenant de l'environnement extérieur, ledit environnement ultranettoyé (24) étant maintenu à une valeur de surpression d'environ 10 Pa, des flux d'air microfiltrés provenant de filtres HEPA de niveau H13 étant utilisés pour générer une telle surpression,
l'étape consistant à stériliser les paraisons moulées (4) se déroulant au moins en partie pendant l'étape de conditionnement thermique des paraisons (4) dans une pluralité de dispositifs de stérilisation (231) configurés pour stériliser au moins intérieurement lesdites paraisons (4), lesdits dispositifs de stérilisation (231) étant installés sur ladite unité de transfert (23), un des dispositifs de stérilisation (231) étant situé en correspondance de chaque poste de support.

2. Procédé selon la revendication 1, dans lequel l'étape de stérilisation des paraisons (4) et l'étape de conditionnement thermique des paraisons (4) sont simultanées.
